# EUROPEAN PATENT APPLICATION

(11) **EP 4 691 466 A1**
(43) Date of publication of application: **11.02.2026**
(21) Application number: 23946380.5
(22) Date of filing: 08.10.2023
(51) Int. Cl.: A61K 31/454, A61K 31/445, A61K 31/55, A61P 27/12

(54) **USE OF PTGDS INHIBITOR IN PREPARATION OF DRUG FOR TREATING CATARACTS**

(30) Priority: 25.07.2023 CN 202310914152
(71) Applicant: The Eye Hospital of Wenzhou Medical University, Wenzhou, Zhejiang 325000 (CN)
(72) Inventor: LI, Jin, Wenzhou, Zhejiang 325000 (CN); LIU, Jiasheng, Wenzhou, Zhejiang 325000 (CN); XU, Yitong, Wenzhou, Zhejiang 325000 (CN); ZHU, Mengchao, Wenzhou, Zhejiang 325000 (CN); SUN, Haisen, Wenzhou, Zhejiang 325000 (CN)
(74) Representative: Furio, Cristina
(86) International application number: PCT/CN2023/123329
(87) International publication number: WO 2025/020299

(57) **Abstract**

The use of a PTGDS inhibitor in the preparation of a drug for treating cataracts. AT-56 is an effective and selective PTGDS inhibitor, and competitively inhibits the generation of PGD2 by occupying a catalytic site of PTGDS, and PTGDS leads to oxidative stress damage of human lens epithelial cells by catalyzing the synthesis of PGD2, thereby promoting the occurrence and development of aging and opacity of a lens, so that AT-56 can effectively alleviate the level of cataracts by means of reducing apoptosis caused by oxidative stress damage.

## Description

### TECHNICAL FIELD

The present invention relates to the technical field of cataract treatment, and in particular to a use of a PTGDS inhibitor in the preparation of a drug for treating cataracts.

### BACKGROUND

Cataracts are partial or whole opacity of lenses, which results in progressive loss of vision. The cataracts are the main cause of blindness. According to statistics, the cataracts account for one-third of all causes of the blindness in China. At the same time, they are also the second major cause of severe visual impairments and moderate visual impairments after uncorrected refractive errors. Among all types of cataracts, age-related cataracts take up the highest proportion. Some large-scale research reports based on populations have shown that a prevalence rate of the age-related cataracts is increased with the increase of age, from 3.9% at 55-64 years old to 92.6% at over 80 years old. With the society's population growth and increased aging population, there will be more patients with the cataracts, which causes a greater burden to individuals and the society. At present, the most effective method for treating the age-related cataracts is surgical treatment, but there is the possibility of intraoperative and postoperative complications such as posterior capsule ruptures, posterior cataracts, and corneal endothelial decompensation. Therefore, a molecular mechanism of aging and opacity of a lens may be understood to provide a potential target for drug treatment for the age-related cataracts. This patent alleviates and delays occurrence and development of the cataracts by developing drugs.

The lens is composed of lens epithelial cells (LECs) and fibroblasts, and the lens epithelial cells are closely attached to an inner surface of an anterior lens capsule. During growth of the lens, after proliferating in a germinal zone of the lens, the lens epithelial cells are differentiated into lens fibroblasts at an equator of the lens. The lens epithelial cells are the most active sites of metabolism in the lens. They play an important role in growth, differentiation and an injury repair of the lens, and in protecting the transparency and the internal environment stability of the entire lens.

A single-cell transcriptome sequencing technology is a new revolutionary technology that has emerged in recent years. Compared with traditional bulk transcriptome sequencing, the single-cell transcriptome sequencing technology can perform an unbiased, repeatable, high-resolution and high-throughput transcriptome analysis on single cells, which can draw a gene expression profile of the single cells. It reveals a regulatory relationship between a complex and rare cell population and genes. The technology determines a disease-related cell population in a comprehensive and unbiased way and can find the effects of the genes on diseases at a cellular level, so that it can be widely applied to disease researches. It is undeniable that the single-cell transcriptome sequencing technology provides an unprecedented opportunity to find targets for treating the diseases.

However, there is no related research on use of the single-cell transcriptome sequencing technology to exploring the molecular mechanism related to aging and opacity of the lens in current literatures. Therefore, this study intends to perform single-cell transcriptome sequencing on the epithelial cells of an aged opaque lens and a transparent lens, acquire significant differential genes and a related pathway between two samples through bioinformatics analysis, and apply an in vitro culture model for the lens epithelial cells and rat in vitro lens organ culture to related verification.

So far, a lanosterol drug has a certain clinical effect on drug treatment for the cataracts.

Through researches on the drug for two congenital cataract families, it is found that lanosterol in a normal lens is the key to regulate abnormal aggregation and depolymerization of lens proteins, which is a great breakthrough in drug treatment for the cataracts. However, as the data does not integrate age-related cataract data, a current clinical experiment also shows some deficiencies, for example, the drug has no significant effect on age-related nuclear cataracts.

### SUMMARY OF THE INVENTION

In order to solve the technical defects of shortage in existing drug treatment for cataracts, the present invention provides use of a PTGDS inhibitor in the preparation of a drug for treating cataracts, capable of delaying and reversing occurrence and development of the cataracts. Technical solutions employed by the present invention are as follows: use of a PTGDS inhibitor in the preparation of a drug for treating cataracts is provided, wherein the PTGDS inhibitor is AT-56.

A concentration of the AT-56 as the PTGDS inhibitor in the drug for treating the cataract is 7-10 µM.

The drug for treating the cataracts is a drug for treating age-related cataracts.

Use of a PTGDS inhibitor being AT-56 in the preparation of a drug for treating an oxidative stress injury of lens epithelial cells is provided.

A concentration of the AT-56 as the PTGDS inhibitor in the drug for treating the oxidative stress injury of the lens epithelial cells is 10 µM.

The present invention has the beneficial effects that the present invention provides the use of the PTGDS inhibitor in the preparation of the drug for treating the cataracts. As an effective and selective PTGDS inhibitor, the AT-56 competitively inhibits production of PGD2 by occupying a catalytic site of PTGDS. The PTGDS catalyzes synthesis of the PGD2 to cause an oxidative stress injury of human lens epithelial cells, thereby promoting occurrence and development of aging and opacity of a lens. By reducing apoptosis caused by the oxidative stress injury, a degree of the cataracts can be effectively reduced.

### BRIEF DESCRIPTION OF THE DRAWINGS

Figure 1A shows overall results of single-cell transcriptome sequencing; Figure 1B shows up-regulated and down-regulated gene expressions of PTGDS in a cataract sample compared with a transparent lens sample, as shown in a violin plot; Figure 1C shows GSEA on 21884 genes through a KEGG database; and Figure 1D is a visualization picture of differential genes in an arachidonic acid metabolism signaling pathway;
Figure 2 is a diagram, in which A shows four epithelial cell subgroups of total samples; and in B-E, a number of each subgroup corresponds to top 10 differential genes of 0-3 lens epithelial cell subgroups;
Figure 3 shows volcano plots (A-D) of differential genes of various subgroups; and E-H show expression levels of PTGDS in various subgroups of two samples;
Figure 4 shows GSEAof arachidonic acid metabolism signaling pathways in various subgroups;
Figure 5 is a visualization diagram of differential genes of arachidonic acid metabolism signaling pathways in various subgroups;
Figure 6 is a diagram, in which A shows relative expression of PTGDS between two samples; B shows expression of the PTGDS in a lens epithelial cell line; C shows expressions of the PTGDS at different AT-56 concentrations; and D shows differences in cell activity between various groups with the addition of different concentrations of AT-56;
Figure 7 is a diagram, in which A shows a control group (DMSO group); and B-E correspondingly show ROS fluorescence staining after the addition of PTGDS with concentrations of 100 pg/mL, 400 pg/mL, 700 pg/mL, and 1000 pg/mL, wherein a scale is as follows: A = B = C = D = E = 50 µM;
Figure 8 is a diagram, in which A shows addition of DMSO; and B-E correspondingly show ROS fluorescence staining after the addition of PGD2 with concentrations of 0.1 nM, 0.4 nM, 0.7 nM, and 1.0 nML, wherein a scale is as follows: A = B = C = D = E = 50 µM;
Figure 9 is a diagram of AT-56 in an H₂O₂ epithelial cell oxidation model capable of inhibiting lens epithelial cells from producing ROS, wherein 9A shows a control group (DMSO group); 9B shows ROS fluorescence staining in an epithelial cell oxidation model with the addition of 200 µM H₂O₂; and 9C shows ROS fluorescence staining with the addition of 200 µM H₂O₂ and 10 µM AT-56, wherein ROS: green fluorescent staining, and a scale is as follows: A = B = C = 100 µM;
Figure 10A shows contents of MDA at different concentrations of PTGDS; Figure 10B shows contents of the MDA at different concentrations of PGD2; and Figure 10C shows a content of the MDA in a case of adding AT-56 to 200 µM H₂O₂;
Figure 11 is a diagram, in which A shows addition of DMSO; B shows addition of 200 µM H₂O₂; and C shows addition of 200 µM H₂O₂ and 10 µM AT-56, wherein red arrows represent apoptotic lens epithelial cells, and a scale is as follows: A = B = C = 50 µM; and
Figure 12 is a diagram, in which A shows a blank control group; B shows treatment with addition of 200 µM H₂O₂ for 12 h; C shows treatment with 200 µm H₂O₂ + 7 µM AT-56 for 12 h; D shows treatment with 200 µm H₂O₂ + 10 µM AT-56 for 12 h; E shows treatment with addition of 200 µM H₂O₂ for 12 h; F shows treatment with 200 µm H₂O₂ for 12 h before treatment with addition of 7 µM AT-56 for 24 h; and G shows treatment with 200 µm H₂O₂ for 12 h before treatment with addition of 10 µM AT-56 for 24 h.

### DETAILED DESCRIPTION OF THE INVENTION

The technical solutions in embodiments of the present invention are clearly and completely described below in combination with the accompanying drawings in the embodiments of the present invention. Apparently, the embodiments described are merely some embodiments rather than all embodiments of the present invention. All other embodiments obtained by those of ordinary skill in the art on the basis of the embodiments of the present invention without making creative efforts fall within the protection scope of the present invention.

In the research of the present invention, it was found that there was a significant difference in a PTGDS gene and its arachidonic acid metabolism pathway between an aged opaque lens and a transparent lens. PTGDS was purified from a rat brain by Urade Y, et al.in 1985. In an arachidonic acid metabolism signaling pathway, as a prostaglandin H2 (PGH2) D-isomerase, PTGDS could catalyze isomerization of a 9, 11-loop endoperoxide group of PGH2 to produce prostaglandin D2 (PGD2) with a 9-hydroxyl group and a 11-keto group. Arachidonic acid (AA) is the most abundant, active and widely distributed polyunsaturated essential fatty acid in a human body. The arachidonic acid has very strong biological activity, and is closely related to oxidative stress. Among organic inhibitors, as an effective and selective PTGDS inhibitor, AT-56 competitively inhibits production of PGD2 by occupying a catalytic site of PTGDS.

### Embodiment 1

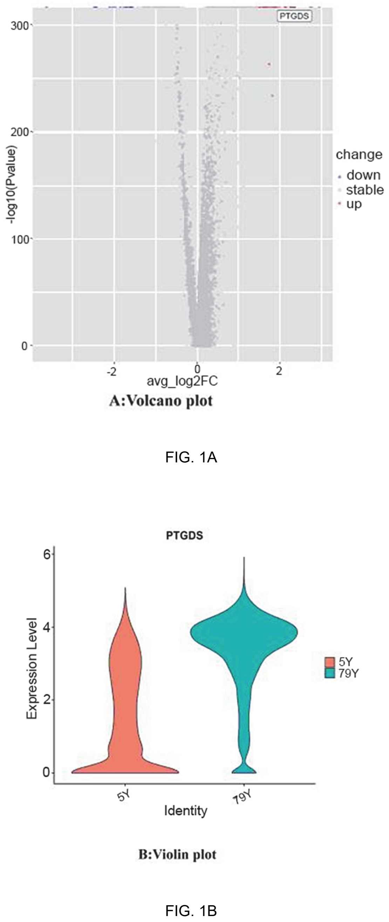

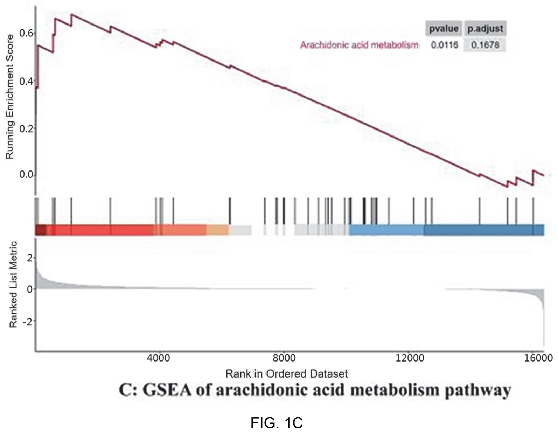

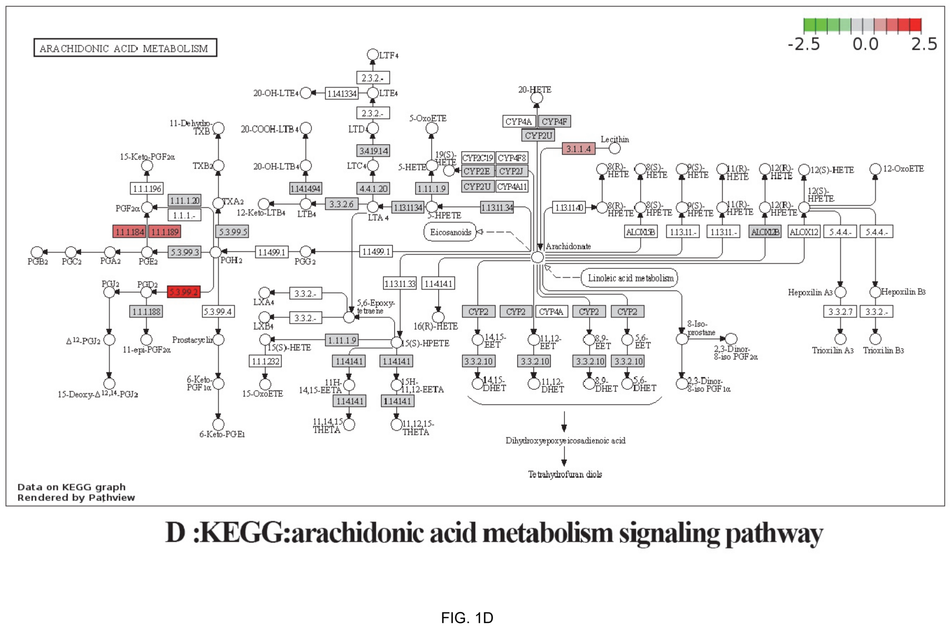

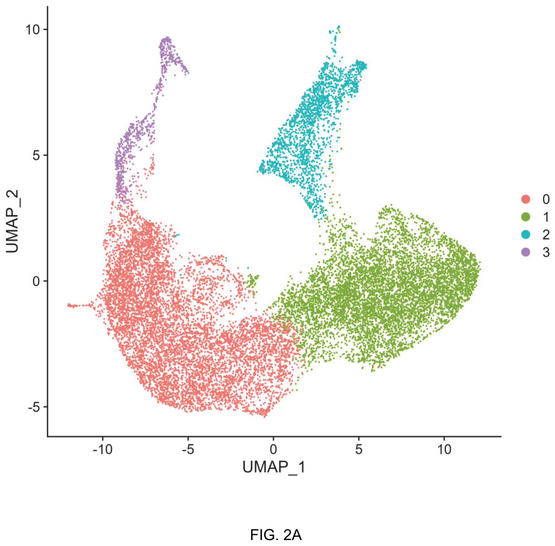

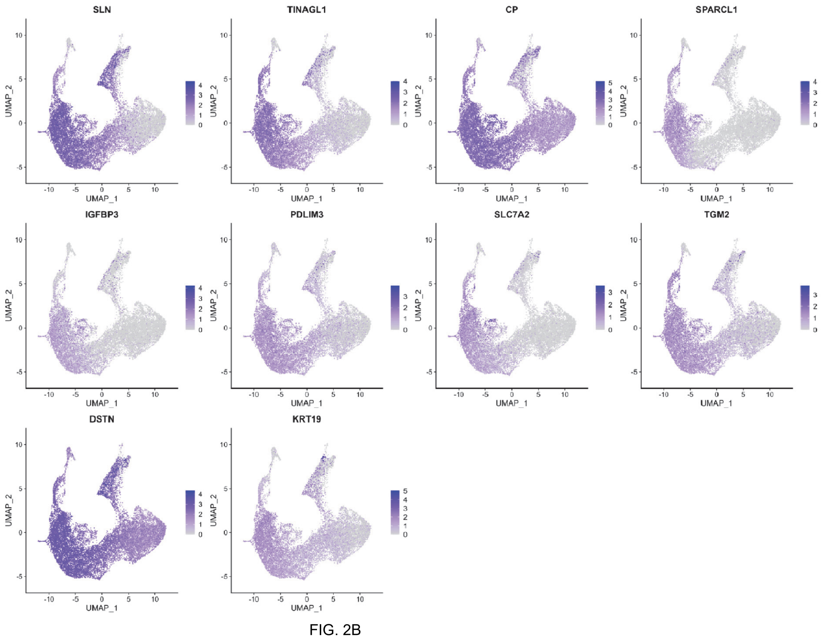

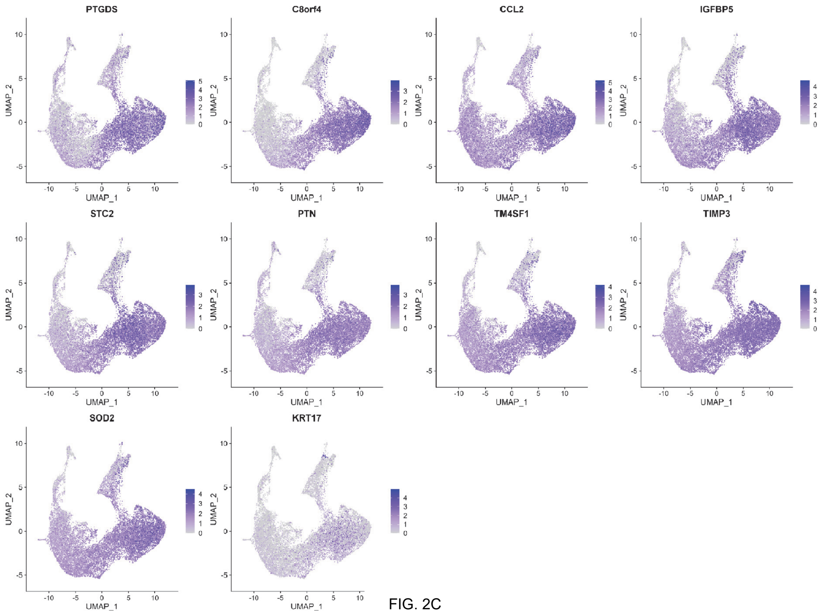

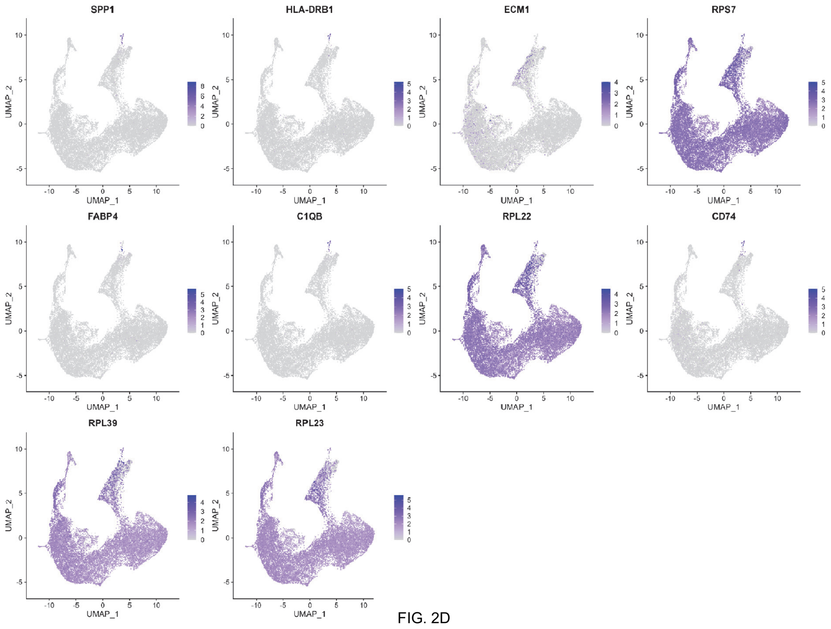

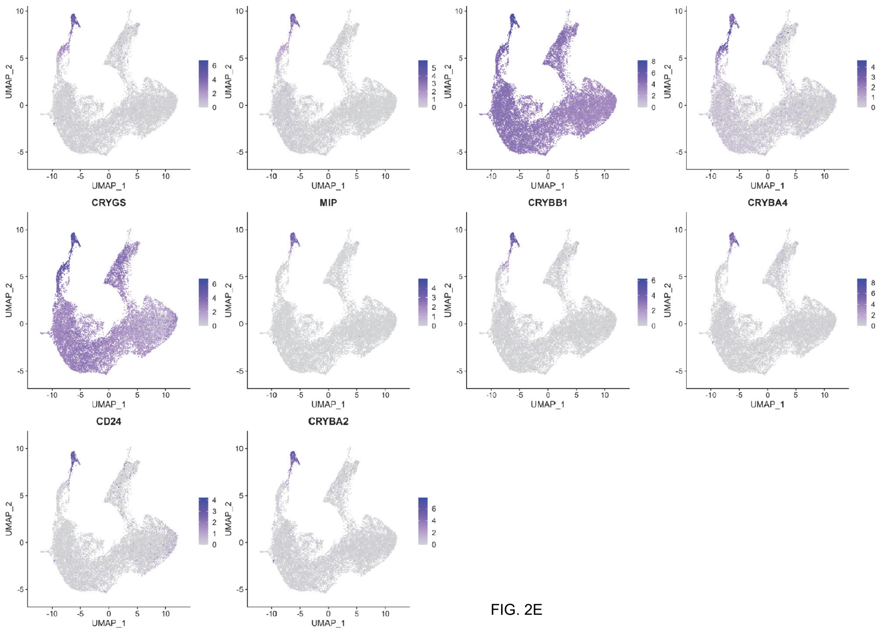

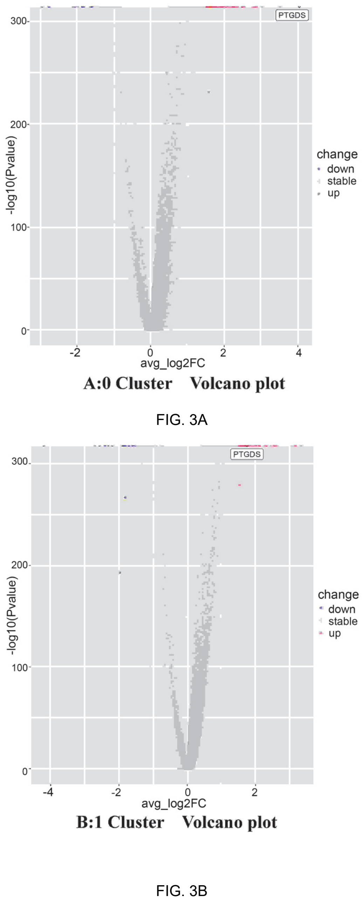

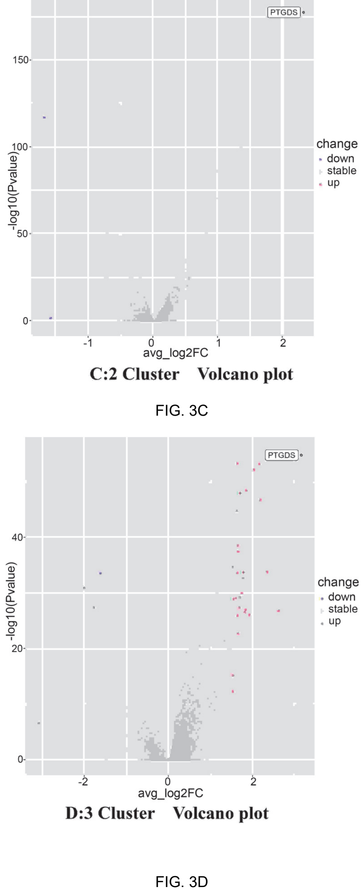

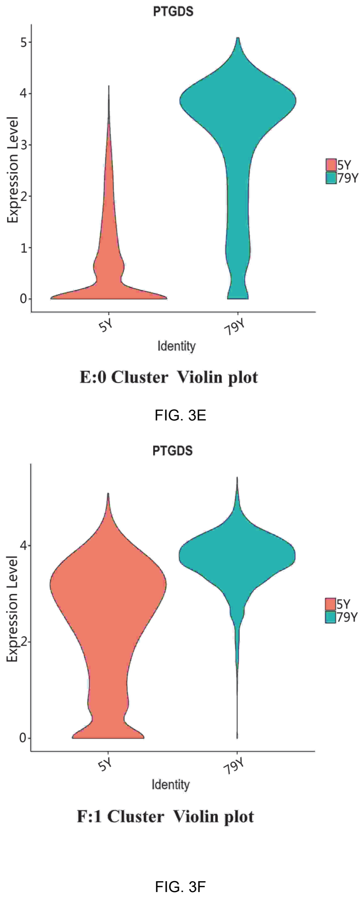

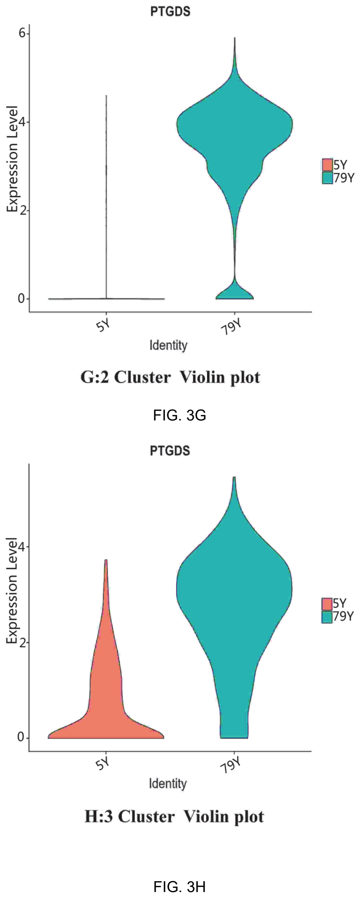

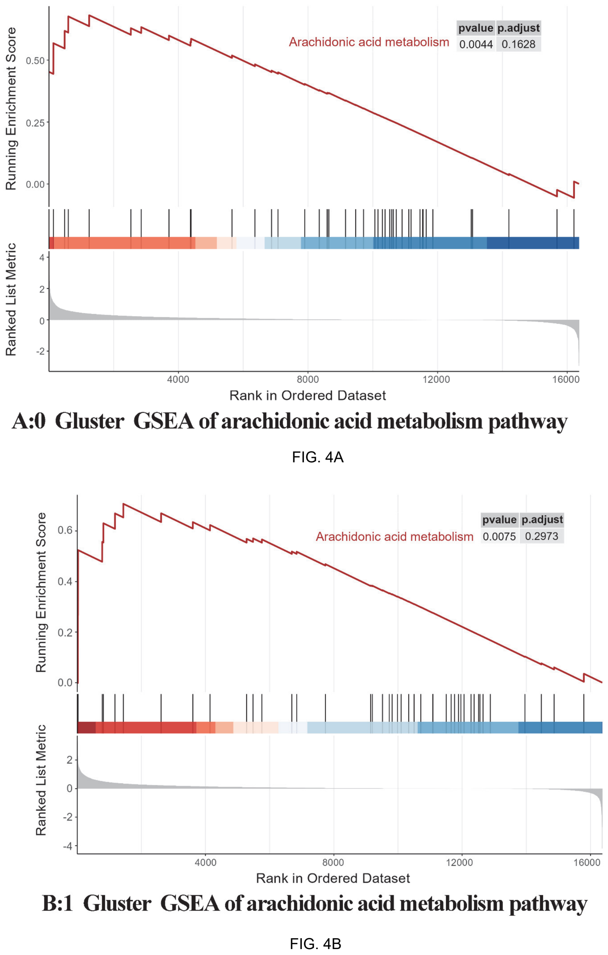

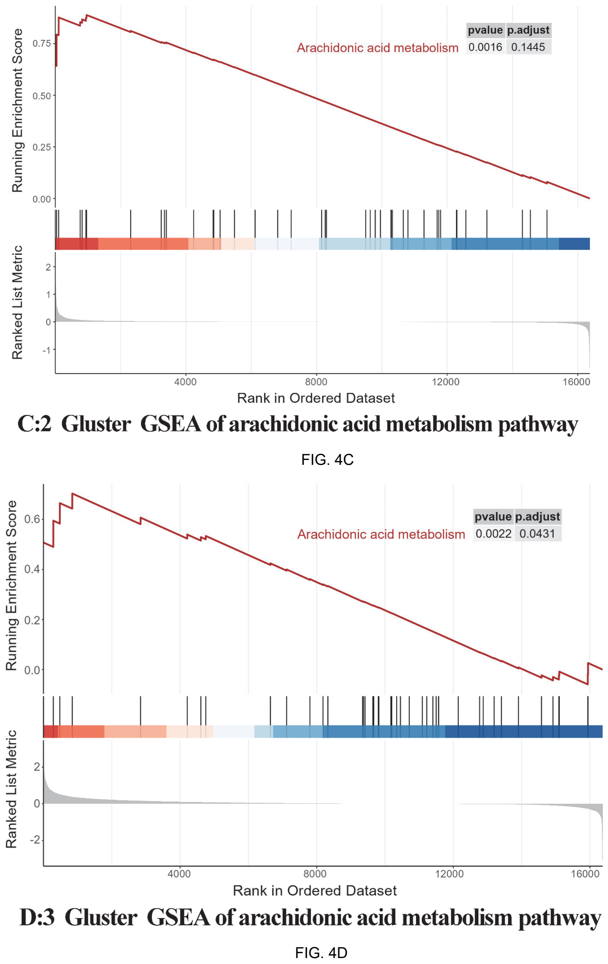

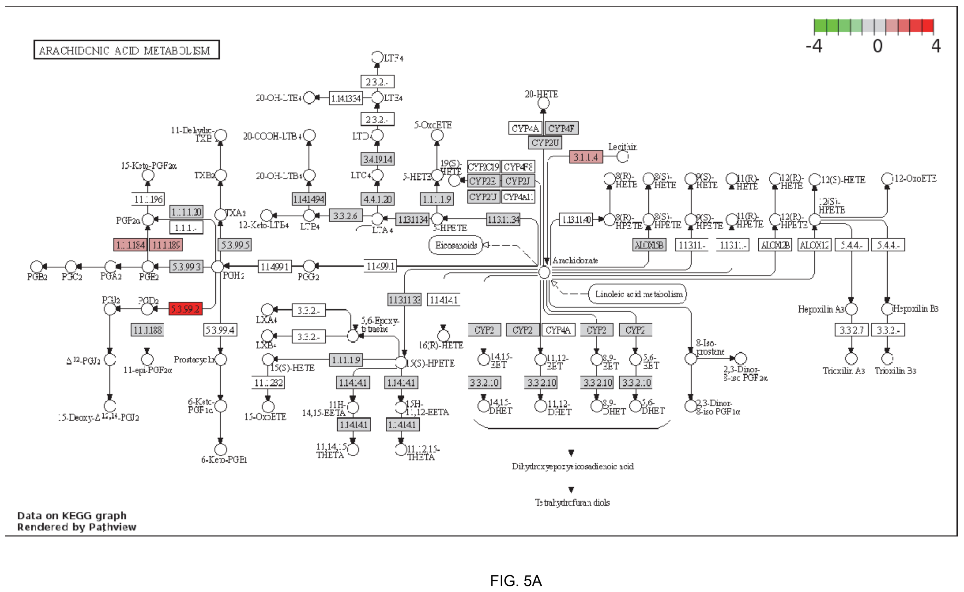

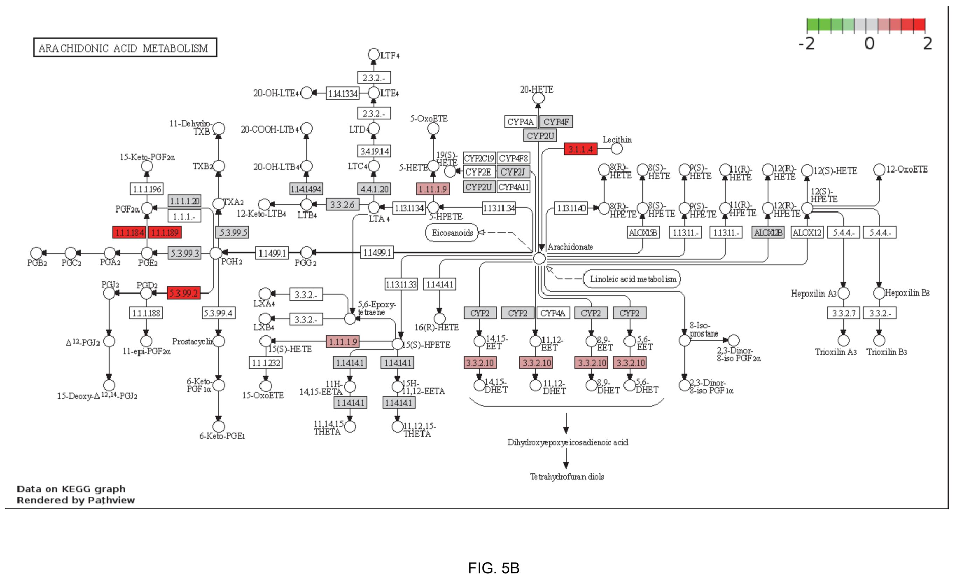

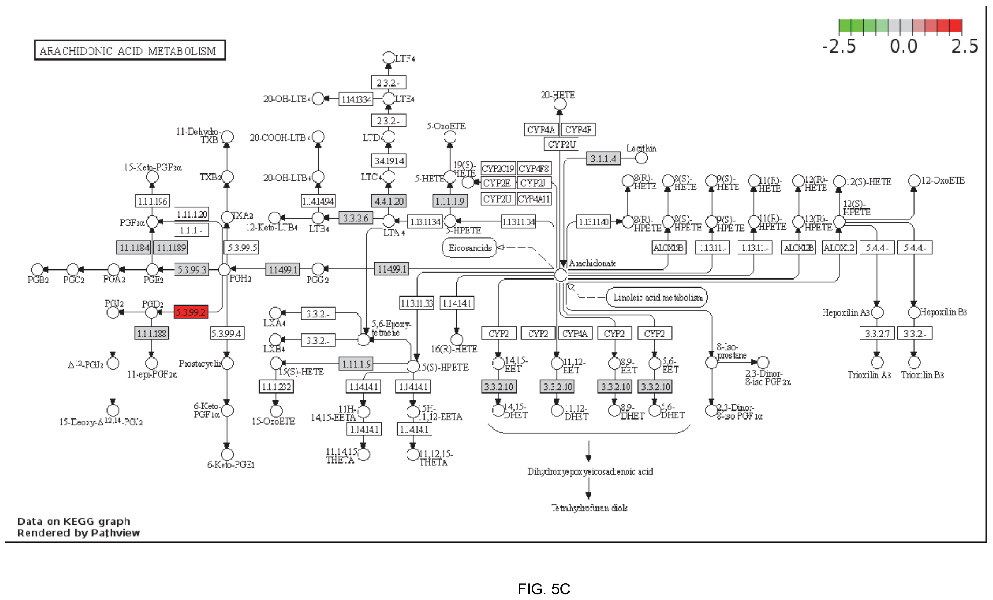

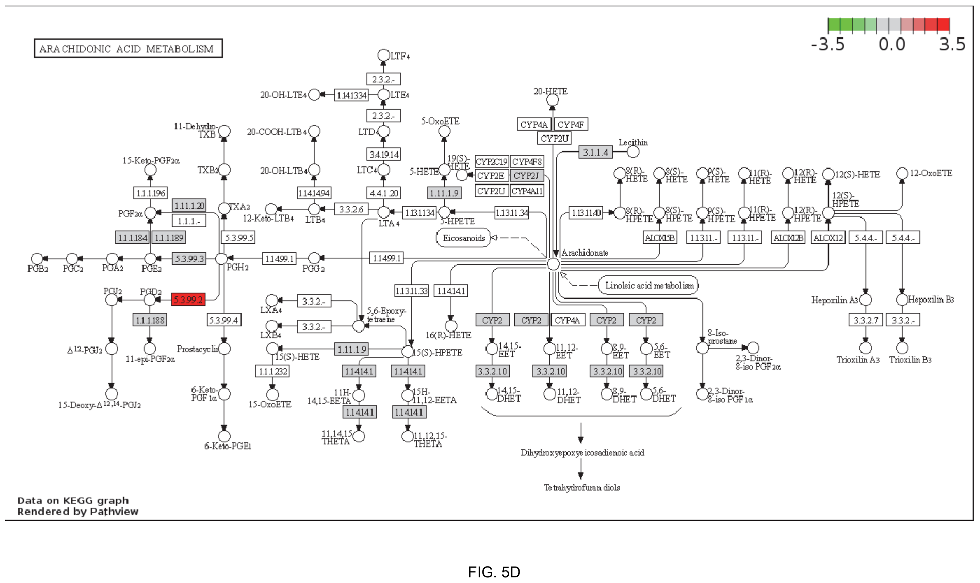

Overall results of single-cell transcriptome sequencing show that among obtained 21884 genes, there are 16537 genes with a value p < 0.05 (Figure 1A). Genes with an absolute value of a fold change between a cataract sample and a normal sample being larger than or equal to 1.5 times were screened: there were 36 genes with log2 (FC) > 1.5 and p < 0.05, which indicated that up-regulated genes of the cataract sample relative to the transparent lens sample were expressed in red; and there were 16 genes with log2 (FC) < -1.5 and p < 0.05, which indicated that down-regulated genes of the cataract sample relative to the transparent lens sample were expressed in blue. Expressions of the PTGDS in the both samples were shown in a violin plot (Figure 1B). Then, GSEA was performed on the 21884 genes through a KEGG database (Figure 1C). It was found that a normative enrichment score (NES) of an arachidonic acid metabolism signaling pathway associated with the PTGDS was 1.52, and the value p was smaller than 0.05, suggesting that aging and opacity of a lens were closely related to this signaling pathway. Visualization of differential genes in the arachidonic acid metabolism signaling pathway was shown in Figure 1D, and it was found that the PTGDS (5.3.99.2) played a role in catalyzing synthesis of the PGD2 in the arachidonic acid metabolism pathway. Through unsupervised cluster analysis, lens epithelial cells were divided into four subgroups (Figure 2). The differential genes in various subgroups were screened according to p < 0.05 and log2 |FC| > 1.5, and it was found that the PTGDS met the above screening criteria in each subgroup (Figure 3). Expressions of the PTGDS in the aged opaque lens sample in various subgroups were all up-regulated compared with the transparent lens sample. Enrichment analysis was performed on genes of the two samples in various subgroups. Results of GSEA show that the arachidonic acid metabolism signaling pathways of the PTGDS in all the subgroups all had the value p smaller than 0.05 and |NES| larger than 1 (Figure 4), suggesting that this pathway might play an important role in pathogenesis of aging and opacity of the lens. The differential genes of the arachidonic acid signaling metabolism pathway in each subgroup were visualized, and the PTGDS (5.3.99.2) could catalyze synthesis of PGD2. Therefore, it is proposed that the PTGDS may cause an oxidative stress injury of human lens epithelial cells by catalyzing synthesis of the PGD2, thereby promoting occurrence and development of aging and opacity of the lens (Figure 5).

### Embodiment 2

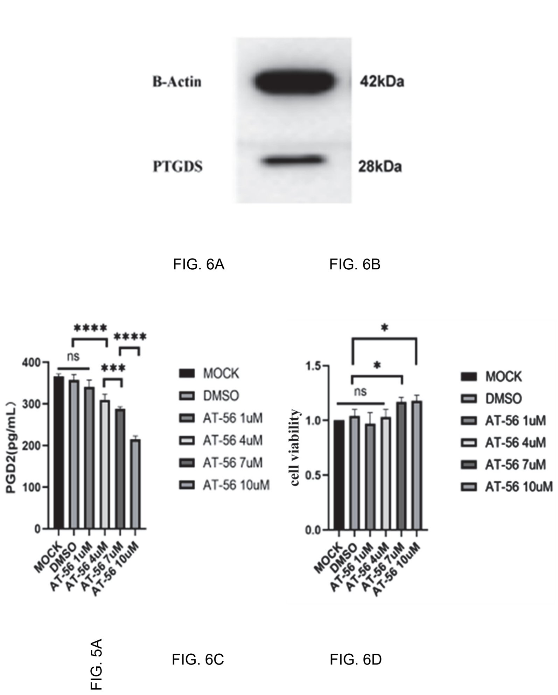

The PTGDS was expressed in both an age-related cataract sample and a transparent lens sample, and an expression level of a PTGDS gene in the former was significantly increased, as shown in Figure 6A. The expression of the PTGDS in an HLEC-B3 cell line of a lens in vitro was confirmed by Western blot (Figure 6B). AT-56 was added to the cell line. It was first found through an ELISA experiment on the PGD2 that 10 µM AT-56 more significantly inhibited production of the PGD2 compared with the AT-56 at other concentrations. Experimental results of CCK8 also further confirmed that the cell activities under the addition of the AT-56 with concentrations of 7 µM and 10 µM were higher than that with the addition of DMSO.

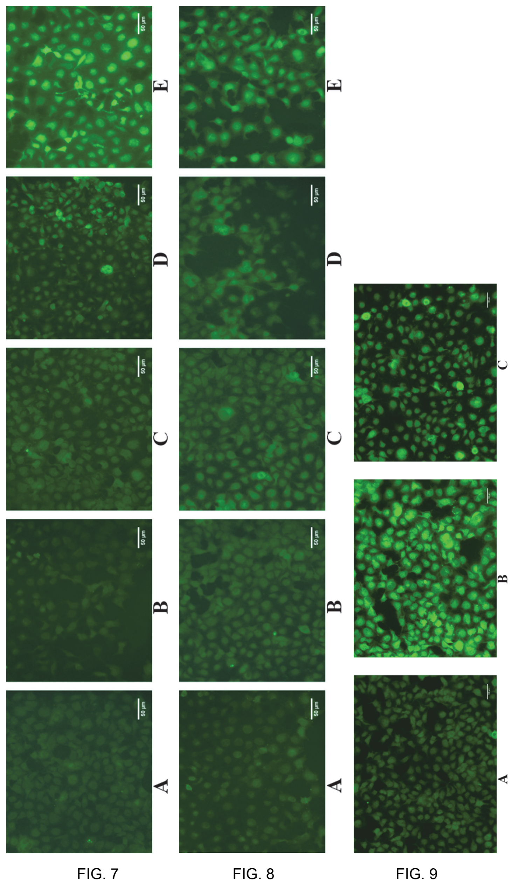

The PTGDS and the PGD2 might both make levels of ROS produced by the lens epithelial cells increased, and were concentration-dependent. As shown in Figure 7, A shows a control group (DMSO group); and B-E correspondingly show ROS fluorescence staining after the addition of PTGDS with concentrations of 100 pg/mL, 400 pg/mL, 700 pg/mL, and 1000 pg/mL, wherein a scale is as follows: A = B = C = D = E = 50µM; As shown in Figure 8, A shows addition of DMSO; and B-E correspondingly show ROS fluorescence staining after the addition of PGD2 with concentrations of 0.1 nM, 0.4 nM, 0.7 nM, and 1.0 nML, wherein a scale is as follows: A = B = C = D = E = 50µM;

The AT-56 in an H₂O₂ epithelial cell oxidation model may inhibit lens epithelial cells from producing ROS (Figure 9), wherein A shows a control group (DMSO group); B shows ROS fluorescence staining in an epithelial cell oxidation model with the addition of 200 µM H₂O₂; and C shows ROS fluorescence staining with the addition of 200 µM H₂O₂ and 10 µM AT-56, wherein ROS: green fluorescent staining, and a scale is as follows: A = B = C = 100µM.

### Embodiment 3

PTGDS/PGD2 makes a content of MDA increased, and the AT-56 effectively reduces production of the MDA.

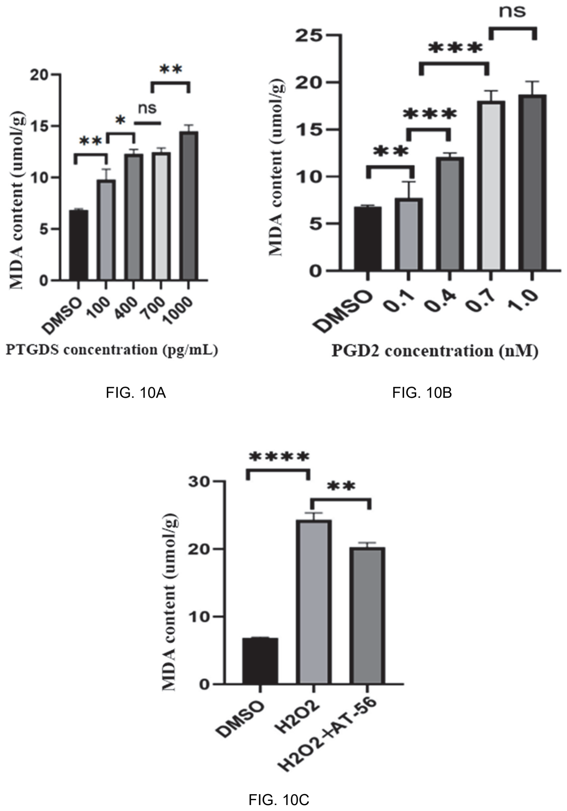

The MDA is an important index of oxidative stress, and measurement on the content of the MDA reflects a degree of an oxidative stress injury. After addition of different concentrations of the PTGDS (Figure 10A) and the PGD2 (Figure 10B), values of the MDA were measured. It was found that the content of the MDA was increased with the increase of the concentration. The addition of the AT-56 to H₂O₂ (Figure 10C) might effectively reduce production of the MDA.

### Embodiment 4

The AT-56 reduces apoptosis caused by the oxidative stress injury.

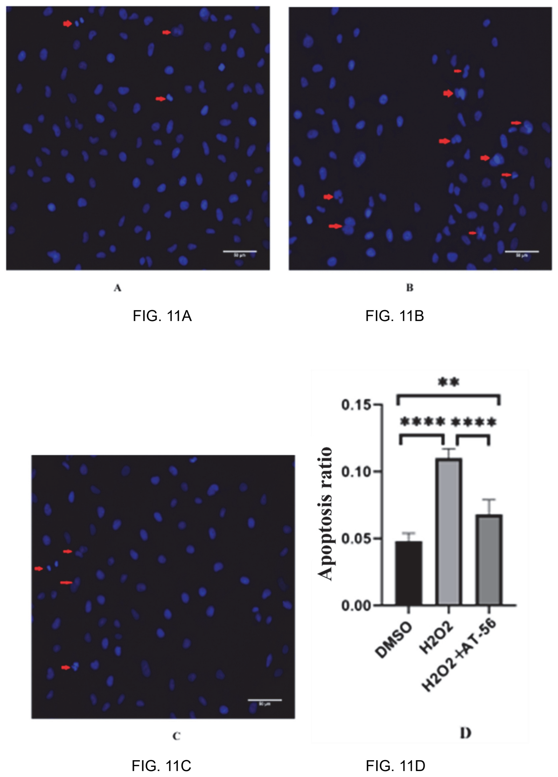

If the lens epithelial cells suffer from the oxidative stress injury, excessive ROS may accumulate in the cells, which can cause apoptosis until generation of cataracts. As shown in Figure 11, addition of 200 µM H₂O₂ to culture of the lens epithelial cells results in apoptosis of more lens epithelial cells. In the other group, addition of 10 µM AT-56 to 200 µM H₂O₂ can effectively reduce apoptosis.

### Embodiment 5

An in vitro oxidative opacity model for a rat lens verified that the AT-56 could alleviate opacity, caused by H₂O₂ oxidative stress, of the lens.

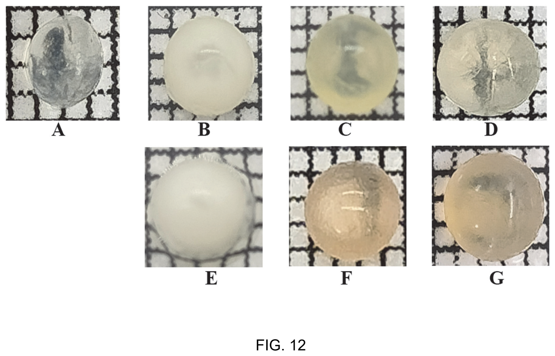

From rat in vitro lens culture, it was found that the addition of the AT-56 alleviated progression of the cataracts (Figures 12A-D). At the same time, after the cataracts were induced with H₂O₂, the addition of the AT-56 could effectively reduce the degree of the cataracts (Figures 12E-G). Notices for those skilled in the art: although the present invention has been described according to the above specific implementations, the invention idea of the present invention is not limited to the present invention, and any modification applying the idea of the present invention will be within the protection scope of the patent right of the present invention.

## Claims

1. A use of a PTGDS inhibitor in preparation of a drug for treating cataracts, wherein the PTGDS inhibitor is AT-56.

2. The use according to claim 1, wherein a concentration of the AT-56 as the PTGDS inhibitor in the drug for treating the cataracts is 7-10 µM.

3. The use according to claim 1, wherein the drug for treating the cataracts is a drug for treating age-related cataracts.

4. An use of AT-56 as a PTGDS inhibitor in preparation of a drug for treating an oxidative stress injury of lens epithelial cells.

5. The use according to claim 4, wherein a concentration of the AT-56 as the PTGDS inhibitor in the drug for treating the oxidative stress injury of the lens epithelial cells is 10 µM.
